# EUROPEAN PATENT APPLICATION

(11) **EP 4 725 387 A1**
(43) Date of publication of application: **15.04.2026**
(21) Application number: 25781985.4
(22) Date of filing: 01.04.2025
(51) Int. Cl.: A61B 1/018

(54) **TREATMENT TOOL INSERTION-USE GUIDE ADAPTER**

(30) Priority: 03.04.2024 JP 2024060274
(71) Applicant: Ort Medical Co., Ltd., Yokohama-shi, Kanagawa, 222-0033 (JP)
(72) Inventor: KARASAWA Koji, Yokohama-shi, Kanagawa 222-0033 (JP); MORI Hirohito, Matsuyama-shi, Ehime 790-0858 (JP)
(74) Representative: Hafner & Kohl PartmbB
(86) International application number: PCT/JP2025/013386
(87) International publication number: WO 2025/211372

(57) **Abstract**

To allow for the smooth insertion of a treatment instrument into a forceps channel via a forceps plug attached to a forceps ferrule of an endoscope. A guide adapter 71 for inserting a treatment instrument of the present invention includes an adapter body 72 including a lubricant storage portion 81 and an insertion portion 82, the lubricant storage portion 81 having one end that is open and an internal space 84 to be filled with a lubricant 75, the insertion portion 82 having an internal space 100 in communication with the internal space 84 and being adapted to be inserted into a forceps plug 31 mounted on a forceps ferrule 21 of an endoscope 10; an inner cover 73 having a slit 107 that allows a treatment instrument to pass through toward the internal space 84, the inner cover 73 being adapted to close the one end of the lubricant storage portion 81; and an outer cover 74 adapted to be attached to the one end of the lubricant storage portion 81 closed by the inner cover 73, and expose the slit 107 provided in the inner cover 73 and a region around the slit 107.

## Description

### Technical Field

The present invention relates to a guide adapter, attached to a plug mounted on a ferrule of an endoscope, for inserting a treatment instrument.

### Background Art

Endoscopes used in the medical field are employed to observe a target location in a body cavity of a patient as a subject (hereinafter sometimes referred to simply as a "target location") and to perform various treatments on the target location using a treatment instrument, by inserting an endoscope insertion portion into the body cavity. An operating handle of the endoscope is provided with a forceps ferrule having a forceps port, for example. The foregoing treatment instrument is inserted into a forceps channel of the endoscope via the forceps port, so that the distal end of the treatment instrument is guided from the distal end of the endoscope insertion portion to the target site within the body cavity.

However, when a target location is observed or various treatments are performed using the endoscope, pressure within the body cavity of the patient may increase. In such a case, there is a risk that a bodily fluid may flow back through the forceps channel of the endoscope, and then leak out from the forceps port of the forceps ferrule. Thus, a forceps plug for covering the forceps port is attached to the forceps ferrule of the endoscope.

Examples of forceps plugs attached to forceps ferrules of endoscopes include a forceps plug having a partition portion therein, and a forceps plug having partition portions in both the body of the forceps plug and a cover mounted on the body of the forceps plug. Such forceps plugs are configured such that when a treatment instrument is inserted through a circular hole provided in each partition portion, the outer periphery of the treatment instrument adheres tightly to the partition portion, creating a liquid-tight seal (for example, see Patent Literature 1).

### Citation List

### Patent Literature

Patent Literature 1: Japanese Patent Laid-Open No. 2017-189304

### Summary of Invention

### Technical Problem

However, when a treatment instrument is fed into the forceps channel through the hole provided in the partition portion of the foregoing forceps plug, there is a problem that frictional force occurs between the partition portion and the outer peripheral surface of the treatment instrument, making it difficult to feed the treatment instrument. Therefore, applying a lubricant in advance to the outer peripheral surface of the treatment instrument is considered effective in reducing the frictional force. However, there is a problem that the task of applying a lubricant in advance before observing a target location or performing various treatments is cumbersome.

An object of the present invention is to provide a guide adapter for inserting forceps that allows for the smooth insertion of a treatment instrument into a forceps channel via a forceps plug attached to a forceps ferrule of an endoscope, without requiring prior application of a lubricant before observing a target location or performing various treatments.

### Solution to Problem

A guide adapter for inserting forceps according to an aspect of the present invention is characterized by including an adapter body including a storage portion and an insertion portion, the storage portion having one end that is open and a first space to be filled with a lubricant, the insertion portion having a second space in communication with the first space and being adapted to be inserted into a plug mounted on a ferrule of an endoscope; a first cover member having a cutout that allows a treatment instrument to pass through toward the first space, the first cover member being adapted to close the one end of the storage portion; and a second cover member adapted to be attached to the one end of the storage portion closed by the first cover member, and expose the cutout provided in the first cover member and a region around the cutout.

The second cover member preferably includes a pressing portion adapted to press the first cover member toward the storage portion when the second cover member is attached to the one end of the storage portion.

The storage portion preferably includes at least one annular protruding portion adapted to bite into the first cover member closing the one end of the storage portion.

The first cover member preferably includes a curved portion adapted to curve toward the first space when the first cover member closes the one end of the storage portion, and the cutout is preferably provided in the curved portion.

The first cover member preferably includes a bent portion formed by combining two wall portions that are adapted to be inclined toward the first space when the first cover member closes the one end of the storage portion, and the cutout is preferably provided at an intersection point of the two wall portions of the bent portion.

### Advantageous Effects of Invention

The present disclosure allows a treatment instrument to be inserted into a forceps channel via a forceps plug attached to a forceps ferrule of an endoscope, without requiring prior application of a lubricant before observing a target location or performing various treatments.

### Brief Description of Drawings

[Fig. 1] Fig. 1 is a schematic explanatory view illustrating a configuration of an endoscope according to this embodiment.
[Fig. 2] Fig. 2 is a perspective view illustrating a configuration around a forceps insertion portion.
[Fig. 3] Fig. 3 is a cross-sectional view illustrating a configuration around the forceps insertion portion.
[Fig. 4] Fig. 4 is an exploded cross-sectional view of the configuration of a guide adapter.
[Fig. 5] Fig. 5 is a cross-sectional view illustrating the procedure for attaching the guide adapter to a forceps plug.
[Fig. 6] Fig. 6 is a cross-sectional view illustrating the procedure for inserting a treatment instrument into a forceps channel via the guide adapter.
[Fig. 7] Fig. 7(a) is a top view illustrating an example of an inner cover having a different slit valve, and Fig. 7(b) is a cross-sectional view along line A-A' in Fig. 7(a).

### Description of Embodiments

Hereinafter, a guide adapter for inserting forceps illustrated in this embodiment will be described using the drawings. In the following description, the guide adapter for inserting forceps may be referred to simply as a "guide adapter." The guide adapter illustrated in this embodiment is attached to a forceps plug mounted on a forceps ferrule of an endoscope.

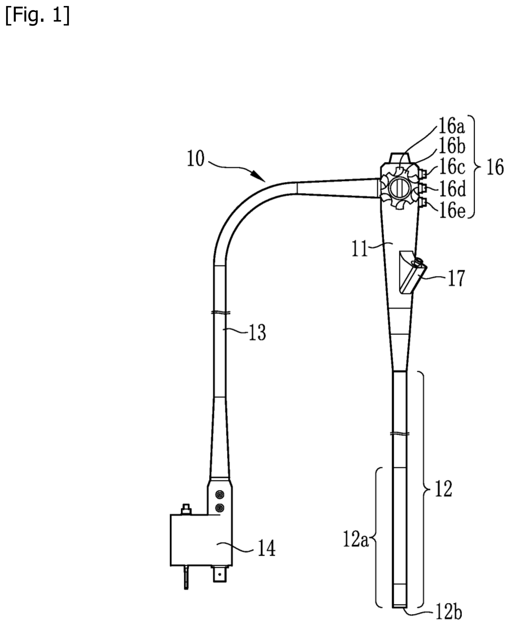

As illustrated in Fig. 1, an endoscope 10 includes an operating handle 11, an endoscope insertion portion 12, a universal tube 13, and a connection portion 14.

The operating handle 11 includes an operating portion 16 and a forceps insertion portion 17. The operating portion 16 includes angle knobs 16a and 16b, an air/water supply button 16c, a suction button 16d, and a shutter button 16e, for example.

The angle knobs 16a and 16b are rotated to perform a bending operation of a curved portion 12a provided on the distal end side of the endoscope insertion portion 12. The angle knob 16a is rotated to bend the curved portion 12a of the endoscope insertion portion 12 in a direction perpendicular to the plane of the paper in Fig. 1, for example. The angle knob 16b is rotated to bend the curved portion 12a of the endoscope insertion portion 12 in the left-right direction in Fig. 1, for example.

The air/water supply button 16c is operated to clean an observation port (not illustrated) or an illumination port (not illustrated) provided in a distal end face 12b of the endoscope insertion portion 12. The suction button 16d is operated to suction dirt in a body cavity through a forceps port (not illustrated) provided at the distal end of the endoscope insertion portion 12. The shutter button 16e is operated to image the inside of the body cavity using an imaging device (not illustrated) provided in the distal end of the endoscope insertion portion 12.

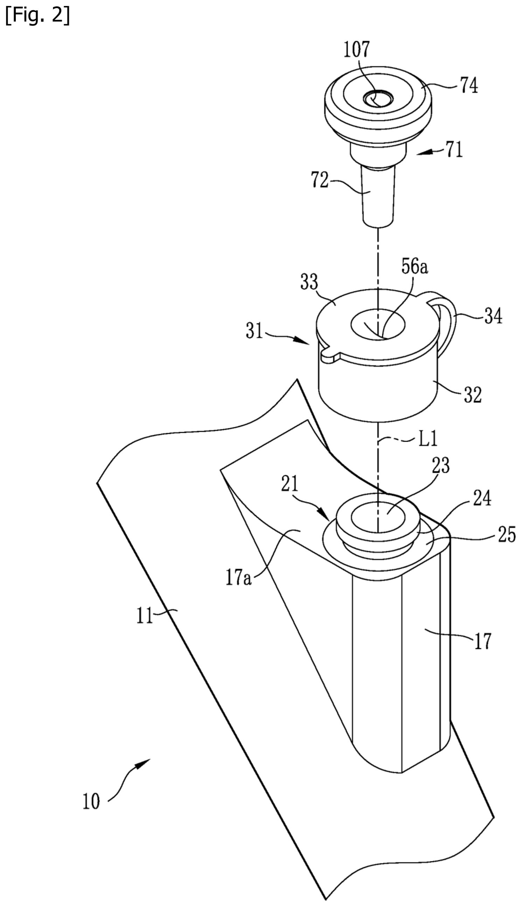

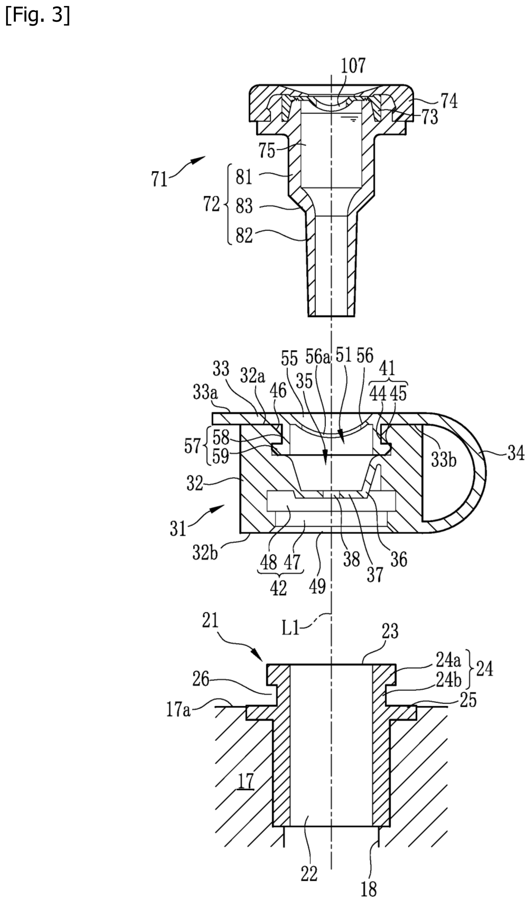

As illustrated in Figs. 2 and 3, the forceps insertion portion 17 is provided with a forceps ferrule 21. The forceps ferrule 21 is formed of a metal material, for example. The forceps ferrule 21 is, for example, a cylindrical member that is open at opposite end portions in the central axis direction of the forceps ferrule 21 (i.e., a direction along a straight line L1 in Figs. 2 and 3). Figs. 2 and 3 each illustrate an example where the central axes of the forceps ferrule 21, a forceps plug 31, and a guide adapter 71 are arranged to be coaxial. In the following description of the forceps ferrule 21, the forceps plug 31, and the guide adapter 71 made with reference to Figs. 2 and 3, their central axis direction may be referred to as a "central axis (L1) direction."

The forceps ferrule 21 is held within the forceps insertion portion 17, with one end in the central axis (L1) direction protruding outward (i.e., in the upward direction in Fig. 2) from an end face 17a of the forceps insertion portion 17. Therefore, of the two end portions of an internal space 22 in the central axis (L1) direction, one end portion protruding (exposed) outward from the end face 17a of the forceps insertion portion 17 serves as a forceps port 23. In addition, the other end portion of the two end portions of the internal space 22 in the central axis (L1) direction, which is located on the opposite side of the one end portion serving as the forceps port 23, is connected to a forceps channel 18 extending to the forceps insertion portion 17. Herein, the forceps ferrule 21 corresponds to a ferrule recited in the claims.

The one end portion, which serves as the forceps port 23, of the forceps ferrule 21 is provided with a to-be-fitted portion 24 adapted to receive the forceps plug 31 (described later) around it. The to-be-fitted portion 24 includes a first annular portion 24a and a second annular portion 24b.

The first annular portion 24a and the second annular portion 24b of the to-be-fitted portion 24 are provided in this order from one end portion toward the other end portion in the central axis (L1) direction. The diameter of the first annular portion 24a is larger than that of the second annular portion 24b.

In addition, the forceps ferrule 21 has a flange portion 25 formed to be continuous with the to-be-fitted portion 24. The diameter of the flange portion 25 is larger than that of the second annular portion 24b of the to-be-fitted portion 24. Thus, as the diameters of the first annular portion 24a and the second annular portion 24b of the to-be-fitted portion 24 and the flange portion 25 are different from one another, a groove 26 is formed on the entire periphery of the forceps ferrule 21 between the first annular portion 24a of the to-be-fitted portion 24 and the flange portion 25. Although Figs. 2 and 3 each illustrate an example where the diameter of the flange portion 25 is larger than that of the first annular portion 24a of the to-be-fitted portion 24, the diameter of the flange portion 25 may be smaller than or equal to that of the first annular portion 24a of the to-be-fitted portion 24.

The forceps plug 31 is formed of a synthetic resin material, such as a general-purpose silicone or styrene-based thermoplastic elastomer, for example. The forceps plug 31 includes a plug body 32, a cover portion 33, and a connection portion 34. The connection portion 34 connects the plug body 32 and the cover portion 33. Herein, the forceps plug 31 corresponds to a plug recited in the claims.

The plug body 32 is a cylindrical member that is open at opposite end portions in the central axis (L1) direction. As the plug body 32 is formed as a cylindrical member, its internal space 35 serves as a first channel. The inside of the first channel 35 is provided with a conical tube portion 36 that partitions the first channel 35. The conical tube portion 36 is open at its upper end and closed at its lower end in the central axis (L1) direction. In addition, the conical tube portion 36 tapers in diameter from a top face 32a of the plug body 32 toward a bottom face 32b of the plug body 32.

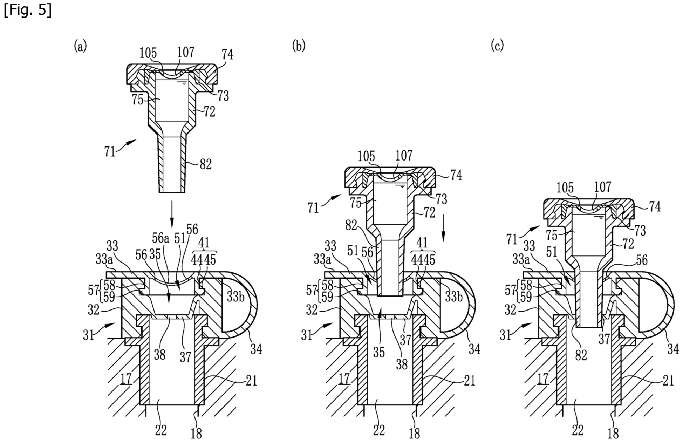

An opening portion 38, which has a circular cross-section in a direction perpendicular to the central axis (L1) direction, for example, is formed in an end face 37 closing the lower end side of the conical tube portion 36. The inside diameter of the opening portion 38 is smaller than the minimum outside diameter of an insertion portion 82 of the guide adapter 71. The insertion portion 82 of the guide adapter 71 inserted into the forceps plug 31 is press-fitted into the opening portion 38. When the insertion portion 82 of the guide adapter 71 is press-fitted into the opening portion 38, a portion (not illustrated) around the opening portion 38 of the end face 37 of the conical tube portion 36 is held in a state where it is in pressure contact with the outer peripheral surface of the insertion portion 82 of the guide adapter 71 (for example, see Fig. 5).

If the inside diameter of the opening portion 38 is too small relative to the minimum outside diameter of the insertion portion 82 of the guide adapter 71, it is anticipated that the insertion portion 82 of the guide adapter 71 cannot be inserted into the opening portion 38. To address such a risk, it is possible to not only provide the opening portion 38 in the end face 37 of the conical tube portion 36, but also provide one or more slits, which extend in the radial direction of the opening portion 38, in the end face 37.

A portion located above the conical tube portion 36, which is provided in the first channel 35, in the central axis (L1) direction serves as a first fitting portion 41 adapted to receive therein a to-be-fitted portion 57 of the cover portion 33. In addition, a portion located below the conical tube portion 36, which is provided in the first channel 35, in the central axis (L1) direction serves as a second fitting portion 42 adapted to receive therein the to-be-fitted portion 24 of the forceps ferrule 21 of the endoscope 10.

The first fitting portion 41 includes, for example, a first opening portion 44 and a second opening portion 45 having an inside diameter larger than that of the first opening portion 44. In the first fitting portion 41, the first opening portion 44 and the second opening portion 45 are provided in this order from the top face 32a side of the plug body 32 in the central axis (L1) direction. Reference numeral 46 denotes a tapered portion that tapers in diameter toward the first fitting portion 41. The tapered portion 46 guides the insertion of the to-be-fitted portion 57 provided in the cover portion 33 into the first fitting portion 41.

The second fitting portion 42 includes, for example, a third opening portion 47 and a fourth opening portion 48 having an inside diameter larger than that of the third opening portion 47. In the second fitting portion 42, the third opening portion 47 and the fourth opening portion 48 are provided in this order from the bottom face 32b side of the plug body 32 in the central axis (L1) direction. Reference numeral 49 denotes a tapered portion that tapers in diameter toward the second fitting portion 42. The tapered portion 49 guides the insertion of the to-be-fitted portion 24 of the forceps ferrule 21 into the second fitting portion 42.

The cover portion 33 has formed therein a second channel 51 for passing a treatment instrument 200. The second channel 51 communicates with the first channel 35 as the cover portion 33 is attached to the plug body 32.

A recess portion 55 is provided at the center of one face 33a of the cover portion 33, the recess portion 55 curving toward a face 33b on the opposite side of the one face 33b. The recess portion 55 and the second channel 51 are separated by a curved wall 56. The curved wall 56 has a slit 56a formed therein. The length of the slit 56a provided in the curved wall 56 is less than the maximum outside diameter of the insertion portion 82 of the guide adapter 71. Consequently, when the insertion portion 82 of the guide adapter 71 is inserted through the slit 56a, the peripheral edge portion of the slit 56a is brought into pressure contact with the outer periphery of the insertion portion 82.

The cover portion 33 has, at the center of its face 33b, the to-be-fitted portion 57 protruding from the face 33b. The to-be-fitted portion 57 is received within the first fitting portion 41 of the plug body 32 when the cover portion 33 is attached to the plug body 32.

The to-be-fitted portion 57 includes a first annular portion 58 and a second annular portion 59. The first annular portion 58 and the second annular portion 59 are provided in this order from the face 33b side. The diameter of the first annular portion 58 is smaller than that of the second annular portion 59. Thus, when the cover portion 33 is attached to the plug body 32, the first annular portion 58 is received within the first opening portion 44 of the first fitting portion 41, and the second annular portion 59 is received within the second opening portion 45 of the first fitting portion 41.

The guide adapter 71 is inserted into the foregoing forceps plug 31, and also, the treatment instrument 200 is inserted through the guide adapter 71 while the guide adapter 71 is inserted into the forceps plug 31. The guide adapter 71 is inserted from the top face 32a side of the plug body 32 while the cover portion 33 of the forceps plug 31 is attached to the plug body 32.

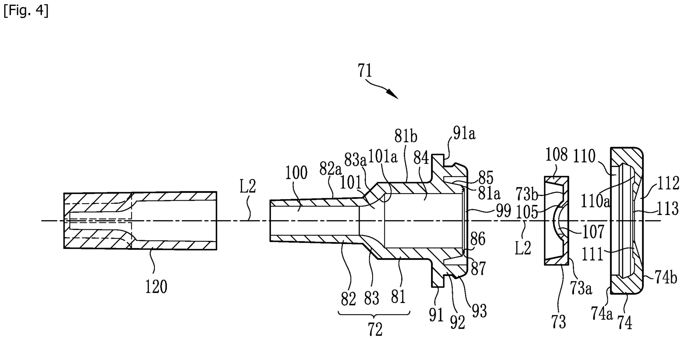

As illustrated in Fig. 4, the guide adapter 71 includes an adapter body 72, an inner cover 73, and an outer cover 74. The inside of the adapter body 72 of the guide adapter 71 is filled with lubricant 75. Herein, the inner cover 73 corresponds to a first cover member recited in the claims, and the outer cover 74 corresponds to a second cover member recited in the claims. In Fig. 4, to describe the configuration of the guide adapter 71, the central axes of the adapter body 72, the inner cover 73, and the outer cover 74 are arranged coaxially. Thus, in the following description of the adapter body 72, the inner cover 73, and the outer cover 74, their central axes may be referred to as a "central axis L2."

The adapter body 72 is formed using a synthetic resin material, such as polycarbonate, for example. The adapter body 72 includes a lubricant storage portion 81, the insertion portion 82, and a connection portion 83. In the adapter body 72, the lubricant storage portion 81, the connection portion 83, and the insertion portion 82 are provided in this order from the right end portion to the left end portion along the central axis (L2) direction of the guide adapter 71 in the drawing.

The lubricant storage portion 81 has a cylindrical shape, for example. The lubricant storage portion 81 is formed by molding a synthetic resin material, for example. The lubricant storage portion 81 has an internal space 84 that is open at one end in the central axis (L2) direction of the adapter body 72. The internal space 84 is filled with the lubricant 75. The outer peripheral surface of one end portion of the lubricant storage portion 81 in the central axis (L2) direction of the guide adapter 71 forms a tapered portion 85 that tapers in diameter toward an end face 81a. Herein, the lubricant storage portion 81 corresponds to a storage portion recited in the claims, and the internal space 84 corresponds to a first space recited in the claims.

The end face 81a of the lubricant storage portion 81 is provided with two annular protruding portions 86 and 87. The two annular protruding portions 86 and 87 bite into an end face 73b of the inner cover 73 when the inner cover 73 is attached to a space 99 of the adapter body 72, thereby maintaining a liquid-tight seal between the lubricant storage portion 81 and the inner cover 73.

A flange portion 91 is provided on the entire periphery of the lubricant storage portion 81 at an end portion of the lubricant storage portion 81 on the opposite side of an end portion continuous with the connection portion 83 in the central axis (L2) direction of the guide adapter 71. The outside diameter of the flange portion 91 is larger than that of the lubricant storage portion 81.

A face 91a of the flange portion 91 is provided with a cylindrical to-be-fitted portion 92. The to-be-fitted portion 92 protrudes toward an end portion where the tapered portion 85 is provided in the central axis (L2) direction of the guide adapter 71. The outer peripheral surface of the to-be-fitted portion 92 is provided with a protrusion 93 protruding in the radial direction of the flange portion 91.

Herein, when the face 91a of the flange portion 91 is assumed as a reference in the central axis (L2) direction of the guide adapter 71, the distance from the face 91a to each of the distal ends of the two annular protruding portions 86 and 87 is shorter than the distance from the face 91a to the distal end of the to-be-fitted portion 92. In addition, the maximum outside diameter of the tapered portion 85 is smaller than the inside diameter of the to-be-fitted portion 92. Thus, the space 99 is formed between the to-be-fitted portion 92 and the tapered portion 85 surrounded by the to-be-fitted portion 92. The inner cover 73 is attached to the space 99.

The insertion portion 82 has a cylindrical shape such that the diameter of the outer peripheral surface of the insertion portion 82 decreases along the central axis (L2) direction of the guide adapter 71, from one end of the insertion portion 82 connected to the connection portion 83 toward the opposite end thereof. That is, the insertion portion 82 has an internal space 100 that is open at opposite end portions in the central axis (L2) direction of the guide adapter 71. Herein, the maximum diameter of the insertion portion 82 is smaller than the outside diameter of the lubricant storage portion 81. In addition, the inside diameter of the insertion portion 82 is larger than the outside diameter of the treatment instrument 200, and is smaller than the inside diameter of the lubricant storage portion 81. The internal space 100 corresponds to a second space recited in the claims.

The connection portion 83 is provided between the lubricant storage portion 81 and the insertion portion 82. As described above, the maximum outside diameter of the insertion portion 82 is smaller than the outside diameter of the lubricant storage portion 81. Thus, an outer peripheral surface 83a of the connection portion 83 forms a tapered surface connecting an outer peripheral surface 81b of the lubricant storage portion 81 and an outer peripheral surface 82a of the insertion portion 82.

The connection portion 83 has an internal space 101 connecting the internal space 84 of the lubricant storage portion 81 and the internal space 100 of the insertion portion 82. An inner peripheral surface 101a of the connection portion 83 is a tapered surface or a curved surface in a cross-section including the central axis (L2) direction of the guide adapter 71.

The inner cover 73 is formed using a synthetic resin material, such as silicone rubber, for example. The inner cover 73 is attached to one end in the central axis (L2) direction of the guide adapter 71, and closes the internal space 84 of the lubricant storage portion 81. The inner cover 73 has a curved portion 105 that is formed at the center of an end face 73a, which is one of two end faces 73a and 73b of the inner cover 73 in the central axis (L2) direction of the guide adapter 71, and curves toward the other end face 73b. The curved portion 105 is provided with a slit 107. The slit 107 corresponds to a cutout in the claims.

When the treatment instrument 200 is not inserted through the slit 107, the curved portion 105 is closed. Meanwhile, when the treatment instrument 200 is inserted through the slit 107, the slit 107 is pushed open by the treatment instrument 200, and adheres tightly to the outer peripheral surface of the treatment instrument 200 inserted through the slit 107. In this manner, when the curved portion 105 of the inner cover 73 is provided with the slit 107, the curved portion 105 serves as a slit valve. The length of the slit 107 is preferably greater than the outside diameter of the treatment instrument 200 inserted therethrough.

The outer peripheral edge portion of the other end face 73b of the inner cover 73 is provided with an outer peripheral wall 108. The outer peripheral wall 108 is held in a state where it is received within the space 99 when the inner cover 73 is attached to the adapter body 72.

The outer cover 74 is formed using a synthetic resin material, such as polypropylene, for example. One end face 74a of the outer cover 74 in the central axis (L2) direction of the guide adapter 71 has a fitting portion 110. The fitting portion 110 is adapted to receive therein the to-be-fitted portion 92 provided in the lubricant storage portion 81. A bottom face 110a of the fitting portion 110 is provided with a protruding portion 111 protruding toward the one end face 74a. The protruding portion 111 is adapted to press the inner cover 73, which is attached to the adapter body 72, toward the adapter body 72 when the outer cover 74 is attached to the adapter body 72. Accordingly, a liquid-tight seal between the adapter body 72 and the inner cover 73 is maintained. Herein, the protruding portion 111 corresponds to a pressing portion recited in the claims.

The outer cover 74 has, at the center of its end face 74b located on the opposite side of the one end face 74a in the central axis (L2) direction of the guide adapter 71, a tapered recess portion 112 that tapers in diameter from the end face 74 toward the fitting portion 110. The recess portion 112 and the fitting portion 110 communicate with each other via a through-hole 113. As the through-hole 113 is provided, the curved portion 105 of the inner cover 73 and the slit 107 provided in the curved portion 105 are exposed when the outer cover 74 is attached to the adapter body 72. Herein, the inside diameter of the through-hole 113 is larger than the outside diameter of the treatment instrument 200 and the length of the slit 107 provided in the inner cover 73.

The lubricant 75 is applied to the surface of the treatment instrument 200 when the treatment instrument 200 is inserted through the guide adapter 71. As the lubricant 75, a gel composed of water, glycerine, a thickener, a pH adjuster, a moisturizer, methylparaben (i.e., an antibacterial agent), and the like, is used.

A cap 120 is attached to the distal end of the insertion portion 82 of the adapter body 72 when the lubricant storage portion 81 of the adapter body 72 is filled with the lubricant 75. Meanwhile, the cap 120 is removed from the insertion portion 82 of the adapter body 72 when the guide adapter 71 is inserted into the forceps plug 31.

With the cap 120 attached to the insertion portion 82 of the adapter body 72 of the guide adapter 71, the lubricant storage portion 81 of the adapter body 72 is filled with the lubricant 75. After the lubricant storage portion 81 of the adapter body 72 is filled with the lubricant 75, the inner cover 73 is attached to the space 99 of the adapter body 72. Finally, the outer cover 74 is attached to the adapter body 72. Accordingly, the formation of the guide adapter 71 is completed.

During the formation of the guide adapter 71, when the inner cover 73 is attached to the adapter body 72, the two annular protruding portions 86 and 87 provided on the end face 81a of the lubricant storage portion 81 of the adapter body 72 bite into the end face 73b of the inner cover 73. Furthermore, when the outer cover 74 is attached to the adapter body 72, the protruding portion 111 of the outer cover 74 presses the inner cover 73, which is attached to the adapter body 72, toward the adapter body 72. Accordingly, a liquid-tight seal between the adapter body 72 and the inner cover 73 is maintained. In such a state, the slit 107 of the inner cover 73 is closed. Thus, the lubricant 75 filling the lubricant storage portion 81 of the adapter body 72 is prevented from leaking through the slit 107.

As illustrated in Fig. 5(a), the forceps plug 31 is attached to the forceps ferrule 21 of the forceps insertion portion 17. In addition, the cover portion 33 of the forceps plug 31 is attached to the plug body 32. Thus, the second channel 51 provided in the cover portion 33 communicates with the first channel 35 provided in the plug body 32. At this time, the slit 56a provided in the cover portion 33 of the forceps plug 31 is closed. Thus, when the endoscope insertion portion 12 of the endoscope 10 is inserted into a body cavity of a patient, pressure within the body cavity of the patient and pressure within the forceps channel 18 of the endoscope 10 inserted into the body cavity of the patient are maintained at a constant level. Thus, even when the pressure within the body cavity of the patient fluctuates and a bodily fluid or the like flows back through the forceps channel 18, the forceps plug 31 attached to the forceps ferrule 21 prevents the bodily fluid or the like from being ejected from the forceps port 23.

For example, when the treatment instrument 200 is used, the guide adapter 71 is inserted into the forceps plug 31. When the insertion portion 82 of the guide adapter 71 presses the slit 56a of the forceps plug 31, the slit 56a opens. Consequently, as illustrated in Fig. 5(b), the insertion portion 82 of the guide adapter 71 is inserted into the forceps plug 31 via the slit 56a.

The distal end of the insertion portion 82 of the guide adapter 71, which has been inserted into the forceps plug 31, presses the end face 37 of the conical tube portion 36. Although not illustrated, as the distal end of the insertion portion 82 of the guide adapter 71 presses the end face 37 of the conical tube portion 36, the diameter of the opening portion 38 provided in the end face 37 of the conical tube portion 36 will increase. Consequently, as illustrated in Fig. 5(c), the insertion portion 82 of the guide adapter 71 is press-fitted into the opening portion 38 from its distal end side. At this time, the outer peripheral surface 82a of the insertion portion 82 press-fitted into the opening portion 38 is brought into pressure contact with the peripheral edge portion of the opening portion 38 in the end face 37 of the conical tube portion 36. That is, the guide adapter 71 is held in a state where it is in pressure contact with each of the end face 37 and the slit 56a of the forceps plug 31. In such a state, even if a bodily fluid or the like flows back through the forceps channel 18, it is possible to prevent the bodily fluid or the like from being ejected from the forceps plug 31 and from the insertion portion of the guide adapter 71 inserted into the forceps plug 31.

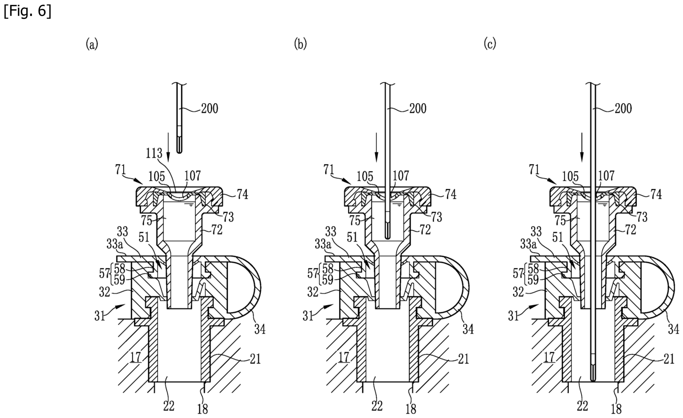

In the guide adapter 71, the slit 107 in the curved portion 105 of the inner cover 73 is exposed through the through-hole 113 of the outer cover 74. As illustrated in Figs. 6(a), 6(b), and 6(c), when the treatment instrument 200 is pushed into the slit 107, the slit 107 is pushed open. Accordingly, the treatment instrument 200 is fed into the forceps channel 18 of the endoscope 10 via the slit 107. That is, the outer peripheral surface of the treatment instrument 200, which has been fed into the guide adapter 71 through the slit 107, moves through the lubricant 75 filling the lubricant storage portion 81 of the guide adapter 71. The treatment instrument 200 is then fed into the forceps channel 18 with the lubricant 75 applied to its outer peripheral surface.

As described above, in the guide adapter 71, the inside diameter of the internal space 100 of the insertion portion 82 is smaller than that of the internal space 84 of the lubricant storage portion 81. The lubricant 75 fills up to the internal space 100 of the insertion portion 82. However, leakage of the lubricant 75 from the distal end of the insertion portion can be prevented by the surface tension acting at the distal end of the insertion portion 82.

In addition, the treatment instrument 200 inserted via the guide adapter 71 is brought into pressure contact with the slit valve 105. That is, while the treatment instrument 200 is inserted through the guide adapter 71, the lubricant 75 held within the guide adapter 71 is prevented from leaking out through the slit 107.

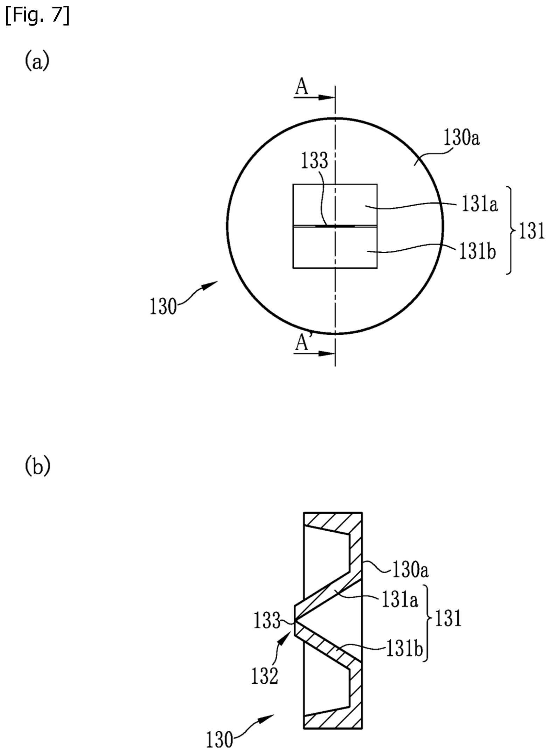

In this embodiment, as the curved portion 105, which curves toward the other end face 73b of the inner cover 73, is provided with the slit 107 at the center of one end face 73a thereof, the curved portion 105 serves as a slit valve. However, the shape of the slit valve is not limited thereto. As illustrated in Figs. 7(a) and 7(b), for example, it is also possible to provide a bent portion 131 formed by combining two wall portions 131a and 131b in a V-shape, at the center of an end face 130a of an inner cover 130, and to form a slit 133 at an intersection point 132 of the two wall portions 131a and 131b.

### <Summary of advantageous effects>

The guide adapter 71 of this embodiment includes the adapter body 72 including the lubricant storage portion 81 and the insertion portion 82, the lubricant storage portion 81 having one end that is open and the internal space 84 to be filled with the lubricant 75, the insertion portion 82 having the internal space 100 in communication with the internal space 84 and being adapted to be inserted into the forceps plug 31 mounted on the forceps ferrule 21 of the endoscope 10; the inner cover 73 having the slit 107 that allows a treatment instrument to pass through toward the internal space 84, the inner cover 73 being adapted to close the one end of the lubricant storage portion 81; and the outer cover 74 adapted to be attached to the one end of the lubricant storage portion 81 closed by the inner cover 73, and expose the slit 107 provided in the inner cover 73 and a region around the slit 107.

Accordingly, there is no need to provide a new forceps plug having a configuration that allows for lubricant filling. In addition, when the guide adapter 71 is attached to the forceps plug 31, it is possible to apply the lubricant 75 to the treatment instrument 200 only by inserting the treatment instrument 200 into the forceps channel 18. Thus, the treatment instrument 200 can be rapidly inserted into the forceps channel 18. In addition, there is no need to apply the lubricant 75 to the treatment instrument in advance for observing a target location in a body cavity or performing various treatments using the endoscope 10. This can simplify the preparation for observing a target location in a body cavity or performing various treatments using the endoscope.

The outer cover 74 preferably includes the protruding portion 111 adapted to press the inner cover 73 toward the lubricant storage portion 81 when the outer cover 74 is attached to one end of the lubricant storage portion 81.

This can maintain a liquid-tight seal between the inner cover 73 and the lubricant storage portion 81.

The lubricant storage portion 81 preferably includes at least one annular protruding portion 86 or 87 adapted to bite into the inner cover 73 that closes one end of the lubricant storage portion 81.

This can maintain a liquid-tight seal between the inner cover 73 and the lubricant storage portion 81.

The inner cover 73 preferably includes the curved portion 105 adapted to curve toward the internal space 84 when the inner cover 73 closes one end of the lubricant storage portion 81, and the slit 107 is preferably provided in the curved portion 105.

This allows for the smooth insertion of the treatment instrument 200 through the slit 107, and also allows a portion around the slit 107 to adhere tightly to the outer peripheral surface of the treatment instrument 200 more easily.

The inner cover 73 preferably includes the bent portion 131 formed by combining the two wall portions 131a and 131b that are adapted to be inclined toward the internal space 84 when the inner cover 73 closes one end of the lubricant storage portion 81. The slit 133 is preferably provided at the intersection point 132 of the two wall portions 131a and 131b of the bent portion 131.

This allows for the smooth insertion of the treatment instrument 200 through the slit 133, and also allows a portion around the slit 133 to adhere tightly to the outer peripheral surface of the treatment instrument 200 more easily.

### Reference Signs List

- 10: endoscope
- 21: forceps ferrule (i.e., ferrule)
- 31: forceps plug (i.e., plug)
- 71: guide adapter for inserting treatment instrument (i.e., guide adapter)
- 72: adapter body
- 73: inner cover (i.e., first cover member)
- 74: outer cover (i.e., second cover member)
- 75: lubricant
- 81: lubricant storage portion (i.e., storage portion)
- 84: internal space (i.e., first space)
- 86, 87: annular protruding portions
- 100: internal space (i.e., second space)
- 105: curved portion
- 107: slit (i.e., cutout)
- 111: protruding portion (i.e., pressing portion)
- 131: bent portion
- 131a, 131b: wall portions
- 132: intersection point of two wall portions 131a and 131b
- 133: slit (i.e., cutout)
- 200: treatment instrument

## Claims

1. A guide adapter for inserting a treatment instrument, **characterized by** comprising:
an adapter body including a storage portion and an insertion portion, the storage portion having one end that is open and a first space to be filled with a lubricant, the insertion portion having a second space in communication with the first space and being adapted to be inserted into a plug mounted on a ferrule of an endoscope;
a first cover member having a cutout that allows a treatment instrument to pass through toward the first space, the first cover member being adapted to close the one end of the storage portion; and
a second cover member adapted to be attached to the one end of the storage portion closed by the first cover member, and expose the cutout provided in the first cover member and a region around the cutout.

2. The guide adapter for inserting a treatment instrument according to claim 1, wherein the second cover member includes a pressing portion adapted to press the first cover member toward the storage portion when the second cover member is attached to the one end of the storage portion.

3. The guide adapter for inserting a treatment instrument according to claim 1, wherein the storage portion includes at least one annular protruding portion adapted to bite into the first cover member closing the one end of the storage portion.

4. The guide adapter for inserting a treatment instrument according to claim 1, wherein:
the first cover member includes a curved portion adapted to curve toward the first space when the first cover member closes the one end of the storage portion, and
the cutout is provided in the curved portion.

5. The guide adapter for inserting a treatment instrument according to claim 1, wherein:
the first cover member includes a bent portion formed by combining two wall portions that are adapted to be inclined toward the first space when the first cover member closes the one end of the storage portion, and
the cutout is provided at an intersection point of the two wall portions of the bent portion.
